# EUROPEAN PATENT APPLICATION

(11) **EP 4 588 453 A1**
(43) Date of publication of application: **23.07.2025**
(21) Application number: 23896066.0
(22) Date of filing: 24.07.2023
(51) Int. Cl.: A61B 34/30

(54) **ROBOTIC MANIPULATOR, JOINT STRUCTURE, AND SURGICAL ROBOT**

(30) Priority: 02.12.2022 CN 202211542727
(71) Applicant: Cornerstone Technology (Shenzhen) Limited, Shenzhen, Guandong 518066 (CN)
(72) Inventor: CHU, Siu Kay, Shenzhen, Guangdong 518066 (CN); CAO, Fengyu, Shenzhen, Guangdong 518066 (CN); LIU, Wo Sang, Shenzhen, Guangdong 518066 (CN); WANG, Zerui, Shenzhen, Guangdong 518066 (CN); TSUI, Yau Ching, Shenzhen, Guangdong 518066 (CN); CHAN, Kwong Sik, Shenzhen, Guangdong 518066 (CN); LAU, Wai Pik, Shenzhen, Guangdong 518066 (CN)
(74) Representative: Balder IP Law, S.L.
(86) International application number: PCT/CN2023/108926
(87) International publication number: WO 2024/113903

(57) **Abstract**

A robotic manipulator, a joint structure, and a surgical robot. The robotic manipulator (100) comprises a base (104), a first link (105), a second link (106), a third link (107), an instrument holder (103), and a transmission mechanism. The first link (105) is rotatable about a first axis (AX1) relative to the base (104). The instrument holder (103) can move a surgical instrument to translate along a second axis (AX2). The second axis (AX2) is fixed relative to the instrument holder (103) and intersects with the first axis (AX1) at a remote center point. The transmission mechanism is used for driving the instrument holder (103) to rotate about a third axis (AX3). The third axis (AX3) passes through the remote center point. The transmission mechanism comprises a pulley assembly and a transmission assembly. The pulley assembly comprises a flange shaft (115) and a pulley (118). The flange shaft (115) comprises a shaft (116) and a flange (117), the flange (117) being connected to the transmission assembly. At a first joint (111), the shaft (116) is connected to the first link (105) and is rotatably adjustable relative to the first link (105). At a second joint (112), the shaft (116) is connected to the second link (106) and is rotatably adjustable relative to the second link (106). The pulley (118) is rotatably sleeved on the shaft (116) and is connected to the transmission assembly.

## Description

### CROSS REFERENCE TO RELATED APPLICATIONS

The present application is based on Chinese patent application No. 2022115427273, entitled "ROBOTIC MANIPULATOR, JOINT STRUCTURE, AND SURGICAL ROBOT", filed on December 2, 2022, and claims the benefit of priority to this Chinese patent application. The entire contents of this Chinese patent application are hereby incorporated into this application by reference.

### TECHNICAL FIELD

The present disclosure relates to the technical field of medical devices, and more specifically to a robotic manipulator, a joint structure, and a surgical robot.

### BACKGROUND

Surgical robots are robots that can be remotely manipulated to complete surgeries, and includes three components: a surgeon console, a patient-side robotic manipulator system and an imaging system. The patient-side robotic manipulator system includes multiple robotic manipulators each having multiple links. Two adjacent links can move relative to each other in a specific degree of freedom, allowing an end of the robotic manipulator to achieve motion in multiple degrees of freedom. The surgical instrument or endoscope is mounted on the end of the robotic manipulator, and during surgery, the surgical instrument passes through tissues such as thoracic wall or abdominal wall to perform surgery instead of a human hand.

The remote center of motion (RCM) is a mechanical design widely used in minimally invasive surgical robotic manipulators, and is defined as access to the patient's abdominal cavity during surgery. Manipulating the robotic manipulator enables the surgical instrument to achieve motion in three degrees of freedom: pitch, yaw, and insertion. During manipulation, the longitudinal axis of the surgical instrument or its extension line keeps passing through the RCM to avoid the surgical instrument from causing non-surgical damage to the patient's abdominal incision.

### SUMMARY

A series of simplified concepts are introduced in the summary section, which will be further detailed in the detailed description section. The summary section of the present disclosure does not attempt to delimit the key features and necessary technical features of the claimed technical solution, nor does it attempt to determine the scope of protection of the claimed technical solution.

Embodiments of the first aspect of the present disclosure provide a robotic manipulator, which includes a base, a first link, a second link, a third link, an instrument holder, and a transmission mechanism.

The first link is connected to the base and is rotatable about a first axis relative to the base. The first axis is fixed relative to the base.

The second link is pivotally connected to the first link at a first joint.

The third link is pivotally connected to the second link at a second joint.

The instrument holder is pivotally connected to the third link, and is configured to hold a surgical instrument and move the surgical instrument along a second axis. The second axis is fixed relative to the instrument holder, and intersects the first axis at a remote center of motion.

The transmission mechanism is configured for driving the instrument holder to rotate around a third axis. The third axis passes through the RCM and is fixed relative to the base.

The transmission mechanism includes pulley assemblies arranged at the first joint and the second joint, and flexible transmission assemblies connecting a dynamic source, the pulley assemblies, and the instrument holder. Each of the pulley assemblies includes: a flanged shaft and a pulley.

The flanged shaft includes a shaft and a flange fixed to each other. The flange is connected to a respective one of the flexible transmission assemblies. The shaft at the first joint is connected to the first link and is rotationally adjustable relative to the first link, and the shaft at the second joint is connected to the second link and is rotationally adjustable relative to the second link.

The pulley is rotatably sleeved on the shaft and connected to a respective one of the transmission assemblies. The pulley at the first joint is fixed to the second link, and the pulley at the second joint is fixed to the third link.

In one embodiment, an end surface of the shaft is provided with a protrusion. The first link and the second link each include a fastener and each define a connection groove corresponding to the protrusion. The protrusion extends into the connection groove, and the connection groove is larger in size than the protrusion. The fastener is configured to fix and adjust a position of the protrusion in the connection groove.

In one embodiment, the first link and the second link each further define an adjustment hole through which the fastener passes. The adjustment hole communicates with the connection groove, and the fastener extends into the connection groove through the adjustment hole to abut against the protrusion.

In one embodiment, the fastener includes a first bolt, and an inner wall defining the adjustment hole is provided with internal threads matching external threads of the first bolt.

In one embodiment, the transmission assemblies include straps including a second strap and a third strap. Two ends of the second strap are respectively connected to the flange at the first joint and the pulley at the second joint. Two ends of the third strap are respectively connected to the flange at the second joint and the instrument holder.

In one embodiment, the transmission mechanism further includes a driving pulley coupled to the dynamic source and driven by the dynamic source to rotate, and the straps further include a first strap two ends of which are respectively connected to the driving pulley and the pulley at the first joint.

In one embodiment, at least one of the first, second, and third straps has two discontinuous segments which are wound in opposite winding directions around the pulley or the driven wheel to drive the pulley or the driven wheel to rotate in two opposite directions, respectively.

In one embodiment, the first strap, the second strap, and the third strap include steel belts.

In one embodiment, at least one of the flange at the first joint, the flange at the second joint, and the driving pulley is provided with a tensioning structure configured to adjust tension of the straps.

In one embodiment, the tensioning structure includes a chamber, a slot, and a bolt. The chamber communicates the outside through the slot, and a movable tensioning block is disposed in the chamber. The bolt abuts against the tensioning block to restrict movement of the tensioning block.

The strap has an end extending into the chamber through the slot and fixed to the tensioning block.

In one embodiment, the bolt includes a second bolt. The flange has an inner wall defining a through hole communicating with the chamber. The inner wall is provided with internal threads mating external threads of the second bolt, and the second bolt extends into the chamber through the through hole to abut against the tensioning block to adjust a position of the tensioning block.

In one embodiment, the bolt includes two second bolts, and the two second bolts respectively abut against two sides of the tensioning block to restrict movement of the tensioning block.

Embodiments of the second aspect of the present disclosure provide a joint structure including: a flanged shaft, a pulley, a first pivot member, and a second pivot member.

The flanged shaft includes a shaft and a flange fixed to each other.

The pulley is pivotally sleeved on the shaft.

The first pivot member is connected to the shaft, and the shaft is rotationally adjustable relative to the first pivot member.

The second pivot member is fixed to the pulley, so that the second pivot member is pivotable relative to the first pivot member.

A flexible transmission assembly is provided inside the second pivot member, the flange is connectable to a rotatable member through the flexible transmission assembly, and the rotatable member rotates relative to the second pivot member with orientation adjustment of the shaft relative to the first pivot member.

In one embodiment, an end surface of the shaft is provided with a protrusion. The first pivot member includes a fastener and defines a connection groove corresponding to the protrusion. The protrusion extends into the connection groove, and the connection groove is larger in size than the protrusion. The fastener is configured to fix and adjust a position of the protrusion in the connection groove.

In one embodiment, the first pivot member is further provided with an adjustment hole through which the fastener passes. The adjustment hole communicates with the connection groove, and the fastener extends into the connection groove through the adjustment hole to abut against the protrusion.

In one embodiment, the fastener includes a bolt with external threads, and an inner wall defining the adjustment hole is provided with internal threads matching the external threads of the bolt.

In one embodiment, the pulley is provided with a tensioning structure including a chamber, a slot, and a bolt. The chamber communicates the outside through the slot, and a movable tensioning block is disposed in the chamber. The bolt abuts against the tensioning block to restrict movement of the tensioning block. The transmission assembly extends into the chamber through the slot and is fixed to the tensioning block.

Embodiments of the third aspect of the present disclosure provide a surgical robot including at least one robotic manipulator as described in the first aspect, or including the joint structure as described in the second aspect.

The details of one or more embodiments of the present disclosure are presented in the following figures and description. Other features, objectives, and advantages of the present disclosure will become apparent from the description, drawings, and claims.

### BRIEF DESCRIPTION OF THE DRAWINGS

The following figures of the present disclosure as part of the present disclosure are for understanding of the present disclosure. The drawings illustrate embodiments of the present disclosure and, together with the description, serve to explain the principles of the present disclosure.

In the accompanying drawings:
Fig. 1 is a schematic diagram of a surgical robot according to an embodiment of the present disclosure;
Fig. 2 is a schematic diagram of a robotic manipulator system according to an embodiment of the present disclosure;
Fig. 3 is a partial structural diagram of a robotic manipulator according to an embodiment of the present disclosure;
Fig. 4 is a partial structural diagram of the robotic manipulator in Fig. 3 in a different state;
Fig. 5 is a partial structural diagram of the robotic manipulator in Fig. 3 in a further different state;
Fig. 6 is a schematic diagram of a robotic manipulator according to an embodiment of the present disclosure, with the first link, second link, and third link omitted;
Fig. 7 is a partially enlarged view of the first joint of the robotic manipulator according to an embodiment of the present disclosure;
Fig. 8 is a schematic diagram of the first joint in Fig. 7, with the first housing removed;
Fig. 9 is a schematic diagram of the first joint in Fig. 8, viewed from another perspective; and
Fig. 10 is a schematic cross-sectional view of the first joint of the robotic manipulator in Fig. 3.

Reference numerals in the drawings are listed as follows:
1 surgical robot; 2 imaging system; 3 control system; 10 robotic manipulator system; 11 pedestal; 12 handle; 13 column; 100 robotic manipulator; 101 adjustment arm; 102 operation arm; 103 instrument holder; 104 base; 105 first link; 106 second link; 107 third link; 108 first strap; 109 second strap; 110 third strap; 111 first joint; 112 second joint; 113 driving pulley; 114 driven wheel; 115 flanged shaft; 116 shaft; 117 flange; 118 pulley; 119 protrusion; 120 connection groove; 121 adjustment hole; 122 first bolt; 123 threaded hole; 124 fastening hole; 125 first housing; 126 chamber; 127 slot; 128 second bolt; 129 tensioning block; AX1 first axis; AX2 second axis; AX3 third axis.

### DETAILED DESCRIPTION OF THE EMBODIMENTS

In the following description, a large number of specific details are presented to provide a more thorough understanding of the present disclosure. However, it is apparent to those skilled in the art that the present disclosure can be implemented without one or more of these details. In other instances, to avoid confusion with the present disclosure, some technical features well known in the art are not described.

It should be noted that the terms used herein are only for describing specific embodiments, and are not intended to limit the exemplary embodiments according to the present disclosure. As used herein, unless the context clearly indicates otherwise, the singular form is also intended to include the plural form. In addition, it should also be understood that when the terms "comprising" and/or "including" are used in this specification, they indicate the presence of the described feature, object, step, operation, element, and/or component, but do not preclude the presence or addition of one or more other features, objects, steps, operations, elements, components, and/or combinations thereof.

The ordinal numbers such as "first" and "second" cited in this disclosure are merely identifiers and do not imply any other meaning, such as a specific order. Moreover, for example, the term "first component" by itself does not imply the existence of a "second component", and the term "second component" by itself does not imply the existence of a "first component" either. It should be noted that the terms "up", "down", "front", "back", "left", "right", "inside", "outside" and similar expressions used in this specification are for illustrative purposes only and are not intended to be restrictive.

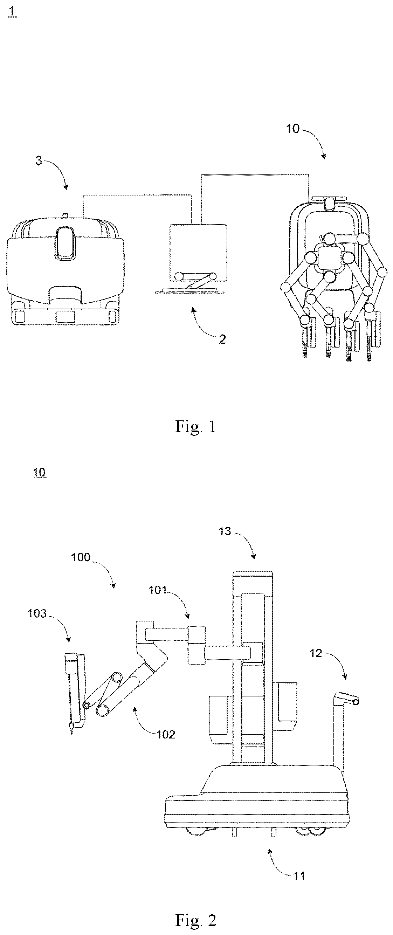

Exemplary embodiments according to the present disclosure are detailed in the following with reference to Figs. 1 to 10.

Referring to Fig. 1, a surgical robot 1 according to embodiments of the present disclosure is a robot that can be remotely manipulated to complete surgeries, which may include a control system 3 (also known as a surgeon console), a robotic manipulator system 10 (also known as a patient-side robotic manipulator system 10), and an imaging system 2.

The control system 3 includes a display unit for displaying the surgical instrument environment, a doctor-manipulated control mechanism, and an armrest. The display unit is provided with an observation window for a doctor to observe, and actions of the doctor-manipulated control mechanism is mapped to actions of the surgical instrument. The armrest is configured to place the doctor's arm. In addition, on the surgeon console, there are other input devices that can be easily touched or pressed by hands or feet to perform various functions for human-computer interaction.

The imaging system 2 has a display screen, an endoscope controller, system electronics, an image processor, and the like.

The robotic manipulator system 10 may include at least one robotic manipulator 100 each having multiple links (such as the first link 105, the second link 106, and the third link 107 introduced in the following). Two adjacent links can move relative to each other in a specific degree of freedom, allowing an end of the robotic manipulator 100 to achieve motion in multiple degrees of freedom (such as seven degrees of freedom, which may vary depending on the surgical instrument). The end of the robotic manipulator 100 is provided with an instrument holder 103, and a surgical instrument or endoscope is detachably mounted on the instrument holder 103.

In one example, referring to Fig. 2, the robotic manipulator system 10 includes a pedestal 11, a column 13 disposed on the pedestal 11, and at least one robotic manipulator 100 disposed on the column 13 (a case of only one robotic manipulator 100 is shown in Fig. 2), The robotic manipulator 100 is movable upward and downward relative to the pedestal 11. The robotic manipulator system 10 may further include a handle 12 disposed on the pedestal 11. An operator may use the handle 12 to facilitate movement of the pedestal 11.

The robotic manipulator 100 may generally include an adjustment arm 101 and an operation arm 102. The instrument holder 103 is provided at an end of the operation arm 102 for holding the surgical instrument or endoscope. The instrument holder 103 may further be provided with an instrument drive device to drive the surgical instrument to perform insertion, clamping, and other actions. Before manipulating the robot for surgery, it is necessary to set up the adjustment arm 101 in such a way that the surgical instrument mounted at the end of the operation arm 102 reaches a designated position, and then lock rotating joints of the adjustment arm 101. During the surgery, surgical operations are performed by remotely controlling the operation arm 102, while the rotating joints of the adjustment arm 101 remain being locked to prevent relative rotation between connecting rods (i.e., links) of the adjustment arm 101 during the operations.

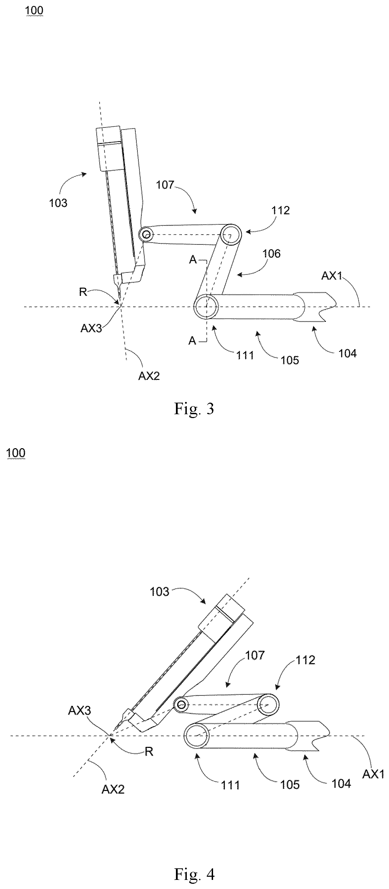

The robotic manipulator 100 of the embodiments of the present disclosure are described in detail below in conjunction with Figs. 3 to 10. The improvements of the robotic manipulator 100 in the present disclosure may involve structural improvements to the aforementioned operation arm 102.

Referring to Figs. 3 to 5, the robotic manipulator 100 according to embodiments of the present disclosure includes a base 104, a first link 105, a second link 106, a third link 107, and an instrument holder 103. The first link 105 is connected to the base 104 and is rotatable about a first axis AX1 relative to the base 104. The first axis AX1 is fixed relative to the base 104. The second link 106 is pivotally connected to the first link 105 at a first joint 111. The third link 107 is pivotally connected to the second link 106 at a second joint 112. The instrument holder 103 is pivotally connected to the third link 107, and is configured to hold a surgical instrument and move the surgical instrument along a second axis AX2. The second axis AX2 is fixed relative to the instrument holder 103, and intersects the first axis AX1 at a remote center R of motion. Rotation of the first link 105 about the first axis AX1 relative to the base 104 cause yaw of the instrument holder 103 about the first axis AX1.

Further, the robotic manipulator 100 further includes a transmission mechanism. The transmission mechanism is configured to drive the instrument holder 103 to rotate about a third axis AX3. The third axis AX3 passes through the remote center R, and the third axis AX3 is fixed relative to the base 104. Therefore, during the operation of the robotic manipulator 100, the surgical instrument mounted on the instrument holder 103 can yaw about the first axis AX1, pitch about the third axis AX3, and move linearly along the second axis AX2, where the first axis AX1, the second axis AX2, and the third axis AX3 intersect at the remote center R. Generally, the instrument holder 103 is such configured that when the surgical instrument is mounted to the instrument holder 103, the longitudinal axis of the surgical instrument coincides with the second axis AX2, i.e., the longitudinal axis of the surgical instrument passes through the remote center R. That is to say, during the operation of the robotic manipulator 100, the longitudinal axis of the surgical instrument or its extension line keeps passing through the remote center R to avoid the surgical instrument from causing non-surgical damage to the patient's abdominal incision.

Further, the transmission mechanism includes pulley assemblies provided at the first joint 111 and the second joint 112, and transmission assemblies connecting a dynamic source, the pulley assemblies, and the instrument holder 103. In the embodiments of the present disclosure, the pulley assemblies and the transmission assemblies can support the robotic manipulator 100, and can also achieve the linkage of the second link 106, the third link 107, and the instrument holder 103.

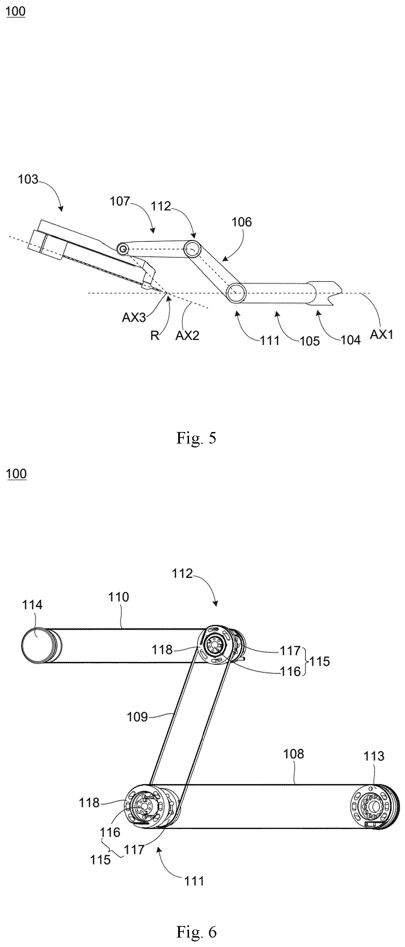

Specifically, referring to Fig. 6, each pulley assembly includes a flanged shaft 115 and a pulley 118. The flanged shaft 115 includes a shaft 116 and a flange 117 fixed to each other. The flange 117 is connected to a respective one of the transmission assemblies. The shaft 116 at the first joint 111 is connected to the first link 105 and is rotationally adjustable relative to the first link 105, and the shaft 116 at the second joint 112 is connected to the second link 106 and is rotationally adjustable relative to the second link 106. The pulley 118 is rotatably sleeved on the shaft 116 and connected to a respective one of the transmission assemblies. The pulley 118 at the first joint 111 is fixed to the second link 106. The pulley 118 at the second joint 112 is fixed to the third link 107. The first joint 111 refers to a joint between the first link 105 and the second link 106, and the second joint 112 refers to a joint between the second link 106 and the third link 107.

For example, referring to Figs. 3 to 5, the transmission mechanism provides transmission from the second link 106 to the third link 107 and further to the instrument holder 103, in such a way that a line (first connection line L1) connecting the rotation axis of the first joint 111 and the rotation axis of the second joint 112, and a line (second connection line L2) connecting the rotation axis of the instrument holder 103 relative to the third link 107 and the rotation axis of the second joint 112, form two adjacent sides of a parallelogram, and the intersection of the two connection lines is a first vertex of the parallelogram. A second vertex, which is diagonal to the first vertex, is located on the third axis AX3.

Generally, the rotation axis of the first joint 111 is the rotation axis which the second link 106 pivots about relative to the first link 105, and the rotation axis of the second joint 112 is the rotation axis which the third link 107 pivots about relative to the second link 106. The two rotation axes of the two joints and the rotation axis of the instrument holder 103 relative to the third link 107 are all parallel to the third axis AX3. That is, the AX3 is perpendicular to the plane of the parallelogram (e.g., the paper surface of Figs. 3 to 5).

Further referring to Fig. 6, during the operation of the robotic manipulator 100, driven by the dynamic source, the transmission assembly drives the pulley 118 at the first joint 111 to rotate, and thus the second link 106 fixed to the first joint 111 pivots, and the flanged shaft 115 at the first joint 111 keeps stationary relative to the first link 105. While the second link 106 pivots, a distance between the rotation axis of the flanged shaft 115 at the first joint 111 and the rotation axis of the pulley 118 at the second joint 112 remains constant. Therefore, a rotation of the pulley 118 at the second joint 112 relative to the flanged shaft 115 at the first joint 111 can be constrained by the transmission assembly. Moreover, as the pulley 118 at the second joint 112 is fixed to the third link 107, an included angle of the third link 107 with the first link 105 remains constant. Further, as the flanged shaft 115 at the second joint 112 is fixed relative to the second link 106, a distance between the rotation axis of the flanged shaft 115 at the second joint 112 and the rotation axis of the instrument holder 103 remains constant while the second link 106 pivots. Therefore, a rotation of the instrument holder 103 relative to the flanged shaft 115 at the second joint 112 can be constrained by the transmission assembly, and an included angle of the instrument holder 103 with the second link 106 remains constant. Thus, the second link 106, the third link 107, and the instrument holder 103 maintain the aforementioned parallelogram during the operation of the robotic manipulator 100, resulting in pitch of the instrument holder 103 about the third axis AX3.

When the intersection point of the first axis AX1 and the second axis AX2 coincides with the second vertex of the aforementioned parallelogram, the first axis AX1, the second axis AX2, and the third axis AX3 intersect at a point, which is determined as the remote center R. For a finished product of the robotic manipulator 100, the remote center R is determined and unique.

In the examples shown in Figs. 3 to 5, the first axis AX1 and the second axis AX2 are arranged to be coplanar with the plane in which the parallelogram is located. However, it will be appreciated that this coplanar relationship is not necessary. In other embodiments not shown, the first axis AX1 and/or the second axis AX2 may intersect with the plane where the parallelogram is located, as long as both the first axis AX1 and the second axis AX2 pass through the remote center R.

In the present disclosure, the transmission assembly connecting the first joint 111 and the second joint 112, and the transmission assembly connecting the second joint 112 and the instrument holder 103 are flexible transmission assemblies (such as straps). The transmission assembly connecting the dynamic source and the first joint 111 may be a flexible transmission assembly (such as one or more strap) or a rigid transmission assembly (including such as one or more transmission rod or multiple gears), which is not limited in the present disclosure.

Generally, during the initial assembly or future maintenance of the robotic manipulator, it is necessary to adjust the flexible transmission assembly to stay in tension. That is because the transmission mechanism works normally only when the flexible transmission assembly is tensioned. The slack of the flexible transmission assembly makes the robotic manipulator unable to meet the requirements of high-precision operation or even fail. However, the inventor found that during tension adjustment of the flexible transmission assembly, the included angle of the third link 107 with the first link 105(which affects the position of the third axis AX3) and/or the included angle of the instrument holder 103 with the second link 106(which affects the position of the second axis AX2) vary accordingly, which results in the first axis AX1, the second axis AX2, and the third axis AX3 not intersecting at the remote center R.

Therefore, in the embodiments of the present disclosure, rotational adjustment of the shaft 116 at the first joint 111 relative to the first link 105 allows the included angle of the third link 107 with the first link 105 to be adjustable, so that the second connection line L2 can be adjusted to be parallel to the line connecting the rotation axis of the first joint 111 and the remote center R, that is, the third axis AX3 can be moved to pass through the remote center R; rotational adjustment of the shaft 116 at the second joint 112 relative to the second link 106 allows the included angle of the instrument holder 103 with the second link 106 to be adjustable, so that the line connecting the intersection point of the first axis AX1 and the second axis AX2 and the rotation axis of the instrument holder 103 relative to the third link 107 can be adjusted to be parallel to the first connection line L1, that is, the second axis AX2 can be moved to pass through the remote center R. As a result, the first axis AX1, the second axis AX2 and the third axis AX3 intersect again at the remote center R.

In one example, referring to Figs. 3 to 5, the first link 105, the second link 106, and the third link 107 are all configured as elongated straight rods. Each straight rod may have a generally rectangular cross-section. The first link 105 and the third link 107 extend in a direction parallel to the first axis AX1. Such configuration can minimize the overall size and manufacturing cost of the robotic manipulator 100 while ensuring its rigidity. In other examples not shown, the first link 105 extend in a direction that is not parallel to the first axis AX1, and/or the third link 107 extend in a direction that is not parallel to the first axis AX1. In other examples not shown, the first link, the second link, and the third link may also be configured into other shapes as needed, such as arc shape, bending shape, or other non-rod shape. The specific shapes of the first link, the second link, and the third link are not limited in the present disclosure.

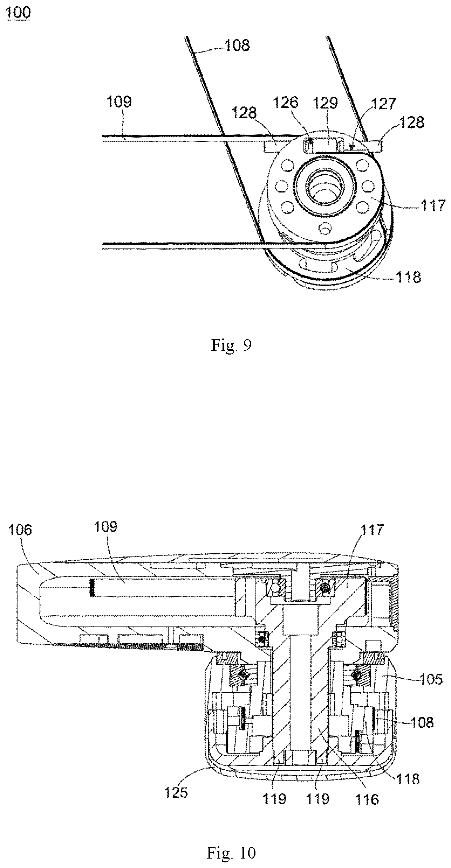

In one example, referring to Fig. 10, the transmission mechanism may be disposed within the first link 105, the second link 106, and the third link 107. Such configuration can protect the transmission mechanism and make the structure of the robotic manipulator 100 more compact. In other examples not shown, the transmission mechanism may alternatively be disposed at other positions of the first, second, and third links, as long as the aforementioned transmission can be achieved.

In one example, to achieve rotational adjustment of the shaft 116 (or flanged shaft 115) relative to the first link 105 or the second link 106, referring to Figs. 6, 7, 8, and 10, a protrusion 119 is provided on an end surface of the shaft 116 of the flanged shaft 115, and the first link 105 and the second link 106 each define a connection groove 120 corresponding to the protrusion 119. The protrusion 119 extends into the connection groove 120, and the connection groove 120 is larger in size than the protrusion 119, so that the protrusion 119 is movable within the connection groove 120. Specifically, the protrusion 119 can rotate, within the connection groove 120, about the rotation axis of the pulley 118 by a certain angle. The first link 105 and the second link 106 each include a fastener for fixing and adjusting the position of the protrusion 119 within the connection groove 120.

Furthermore, the first link 105 and the second link 106 each further define an adjustment hole 121 through which the fastener passes. The adjustment hole 121 communicates with the connection groove 120, and the fastener extends into the connection groove 120 through the adjustment hole 121 to abut against the protrusion 119, thereby limiting the position of the protrusion 119 within the connection groove 120.

Further, the fastener includes a first bolt 122, and an inner wall defining the adjustment hole 121 is provided with internal threads matching external threads of the first bolt 122. Screwing the first bolt 112 can adjust the position of the protrusion 119 within the connection groove 120.

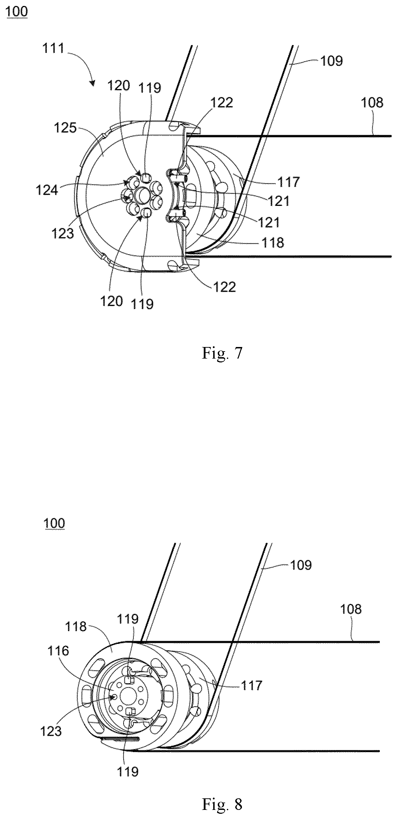

Taking the first joint 111 as an example, referring to Figs. 7 and 8, the end surface of the shaft 116 of the flanged shaft 115 is provided with two aforementioned protrusions 119 spaced from each other in the circumferential direction of the shaft 116. The first link 105 includes a first housing 125, and the transmission assembly is disposed within the first housing 125. A front surface, facing the shaft 116, of the first housing 125 defines two connection grooves 120. The fasteners include two first bolts 122 for adjustment of the two protrusions 119, respectively. A side surface of the first housing 125 defines two adjustment holes 121 to allow the two first bolts 122 to be screwed in, respectively, for adjustment of the protrusions 119 in two opposite rotational directions. Referring to Fig. 10, the pulley 118 is rotatably disposed in the first link 105, and the flange 117 of the flanged shaft 115 is rotatably disposed in the second link 106. The shaft 116 of the flanged shaft 115 extends out of the second link 106 and into the first link 105 to be rotatably connected to the pulley 118.

In one example, when the protrusion 119 is located at an appropriate position in the connection groove 120, the fastener may further include at least one third bolt (not shown in the figure) to fix the flanged shaft 115 to the first link 105. Correspondingly, the shaft 116 defines at least one threaded hole 123 matching the third bolt. The threaded hole 123 extends parallel to the axial direction of the shaft 116. The threaded hole 123 and the protrusion 119 are spaced from each other in the circumferential direction of the shaft 116. Correspondingly, the front surface of the first housing 125 further define multiple fastening holes 124, and each fastening hole 124 is larger in size than the threaded hole 123. Each fastening hole 124 may be designed to have an oblong or racetrack shape, so that the threaded hole 123 would not be covered by the first housing 125 in spite of the position change of the threaded hole 123 during rotational adjustment of the flanged shaft 115. After the rotational adjustment of the flanged shaft 115 is finished, the third bolt is inserted into the fastening hole 124 and the threaded hole 123 to fix the shaft 116 of the flanged shaft 115 to the first housing 125, so as to avoid a relative movement of the two in the axial direction.

In one example, referring to Fig. 10, the first housing 125 may include an inner housing and an outer housing. The inner housing defines the aforementioned connection groove 120, adjustment hole 121, and fastening hole 124. The outer housing is detachably fixed to the inner housing to cover and protect the inner housing.

In one example, the transmission assembly connecting the dynamic source and the first joint 111, the transmission assembly connecting the first joint 111 and the second joint 112, and the transmission assembly connecting the second joint 112 and the instrument holder 103 are all flexible transmission assemblies. For example, referring to Fig. 6, the flexible transmission assemblies include straps including a first strap 108, a second strap 109, and a third strap 110. The first strap 108 connects the dynamic source and the pulley assembly at the first joint 111, the second strap 109 connects the pulley assembly at the first joint 111 and the pulley assembly at the second joint 112, and the third strap 110 connects the pulley assembly at the second joint 112 and the instrument holder 103. For example, the first strap 108, the second strap 109, and the third strap 110 may include steel belts. The steel belt may be of a single-layer structure or a multi-layer laminated structure.

Furthermore, the transmission mechanism further includes a driving pulley 113 and a driven wheel 114. The first strap 108 has one end connected to the dynamic source through the driving pulley 113, and one other end connected to the pulley 118 at the first joint 111. The second strap 109 has two ends respectively connected to the flange 117 at the first joint 111 and the pulley 118 at the second joint 112. The third strap 110 has one end connected to the flange 117 at the second joint 112, and one other end connected to the instrument holder 103 through the driven wheel 114. In other alternative examples, the transmission mechanism may not include the first strap 108 and the driving pulley 113, and the transmission assembly connecting the dynamic source and the first joint 111 may be a rigid transmission assembly, including such as one or more transmission rod or multiple gears. In other alternative examples, the transmission mechanism may not include the driven wheel 114, and the other end of the third strap 110 be connected to the instrument holder 103 directly or through other intermediate parts.

Further, at least one of the first strap 108, the second strap 109, and the third strap 110 has two discontinuous segments which wrap in opposite winding directions around the pulley 118 or the driven wheel 114 to drive the pulley 118 or the driven wheel 114 to rotate in two opposite directions, respectively. Such configuration can improve the transmission accuracy of the transmission mechanism on the one hand, and facilitate the tension adjustment of the straps on the other hand.

The tension adjustment of the straps can be achieved through the respective tensioning structures. The tensioning structure may be provided on the pulley assembly. For example, at least one of the flange 117 at the first joint 111, the flange 117 at the second joint 112, and the driving pulley 113 is provided with the tensioning structure. The tensioning structure may be provided on the flexible transmission assembly. For example, at least one of the first strap 108, the second strap 109, and the third strap 110 is provided with the tensioning structure. As an illustrative example, referring to Figs. 6 and 9, the flange 117 at the first joint 111, the flange 117 at the second joint 112, and the driving pulley 113 are each provided with the tensioning structure, which allows for the tension adjustment of the first strap 108, the second strap 109, and the third strap 110.

In one example, the tensioning structure may include a chamber 126, a slot 127, and a bolt. The chamber 126 communicates the outside through the slot 127, and a movable tensioning block 129 is disposed in the chamber 126. The bolt abuts against the tensioning block 129 to restrict movement of the tensioning block 129. The strap has an end extending into the chamber 126 through the slot 127 and fixed to the tensioning block 129. As a result, the position of the tensioning block 129 can be adjusted via the bolt, so that the slack strap can be tensioned again.

In one example, the bolt includes a second bolt 128. The flange 117 has an inner wall defining a through hole communicating with the chamber 126. The inner wall is provided with internal threads mating external threads of the second bolt 128, and the second bolt 128 extends into the chamber 126 through the through hole to abut against the tensioning block 129 to adjust a position of the tensioning block 129.

Specifically, referring to Fig. 9, the bolt may include two second bolts 128, and the two second bolts 128 are respectively abutted against two sides of the tensioning block 129 to restrict the movement of the tensioning block 129. In this way, movements of the tensioning block 129 in two directions can be restricted, so that the tensioning block 129 can be stably fixed in a desired position and keep in position without sliding out of the chamber 126 when the strap is slack, thereby improving safety. In this embodiment, the tensioning structures of the flange 117 at the first joint 111, the flange 117 at the second joint 112, and the driving pulley 113 are all in the aforementioned configuration. In alternative embodiments, the tensioning structures of the flange 117 at the first joint 111, the flange 117 at the second joint 112, and the driving pulley 113 may have different configurations. The present disclosure does not limit the position and specific form of the tensioning structure, as long as it can achieve the adjustment of the tension of the strap.

Another aspect of the present disclosure provides a joint structure, which includes a flanged shaft 115, a pulley 118, a first pivot member, and a second pivot member. The flanged shaft 115 includes a shaft 116 and a flange 117 fixed to each other. The pulley 118 is pivotally sleeved on the shaft 116. The first pivot member is connected to the shaft 116, and the shaft 116 is rotationally adjustable relative to the first pivot member. The second pivot member is fixed to the pulley 118, so that the second pivot member is pivotable relative to the first pivot member.

A flexible transmission assembly is provided inside the second pivot member, the flange 117 is connectable to a rotatable member through the flexible transmission assembly, the rotatable member rotates relative to the second pivot member with orientation adjustment of the shaft relative to the first pivot member.

In one example, an end surface of the shaft 116 is provided with a protrusion 119. The first pivot member includes a fastener and defines a connection groove 120 corresponding to the protrusion 119. The protrusion 119 extends into the connection groove 120, and the connection groove 120 is larger in size than the protrusion 119. The fastener is configured to fix and adjust a position of the protrusion 119 in the connection groove 120.

In one example, the first pivot member is further provided with an adjustment hole 121 through which the fastener passes. The adjustment hole 121 communicates with the connection groove 120, and the fastener extends into the connection groove 120 through the adjustment hole 121 to abut against the protrusion 119.

In one example, the fastener includes a bolt with external threads, and an inner wall defining the adjustment hole 121 is provided with internal threads matching the external threads of the bolt.

In one example, the pulley is provided with a tensioning structure for tension of the flexible transmission assembly. The tensioning structure may include a chamber 126, a slot 127, and a bolt. The chamber 126 communicates the outside through the slot 127, and a movable tensioning block 129 is disposed in the chamber 126. The bolt abuts against the tensioning block 129 to restrict movement of the tensioning block 129. The transmission assembly extends into the chamber 126 through the slot 127 and is fixed to the tensioning block 129.

In one example of the present disclosure, for example, at the first joint 111, the first pivot member may be the first link 105, and the second pivot member may be the second link 106. In another example of the present disclosure, for example, at the second joint 112, the first pivot member may be the second link 106, and the second pivot member may be the third link 107.

The surgical robot provided in the embodiments of the present disclosure has the aforementioned robotic manipulator or joint structure, and therefore has similar technical effects to the aforementioned robotic manipulator or joint structure, which will not be further described here.

Unless otherwise defined, the technical and scientific terms used in this paper have the same meaning as generally understood by those skilled in the art of the present disclosure. The terms used in this paper are only for purposes of describing specific implementations and are not intended to limit the scope of the present disclosure. Features described in one embodiment herein may be applied to another embodiment individually or in combination with other features, unless the feature is not applicable in the other embodiment or otherwise specified.

The present disclosure has been described through the above embodiments, but it should be understood that the above embodiments are only for the purposes of illustration and explanation, and the present disclosure is not limited to the above embodiments. According to the teachings of the present disclosure, more variations and modifications can be made, and these variations and modifications all fall within the scope of protection claimed by the present disclosure.

## Claims

1. A robotic manipulator, comprising:
a base;
a first link connected to the base and is rotatable about a first axis relative to the base, wherein the first axis is fixed relative to the base;
a second link pivotally connected to the first link at a first joint;
a third link pivotally connected to the second link at a second joint;
an instrument holder pivotally connected to the third link and configured to hold a surgical instrument and move the surgical instrument along a second axis, wherein the second axis is fixed relative to the instrument holder, and intersects the first axis at a remote center of motion, RCM; and
a transmission mechanism configured for driving the instrument holder to rotate around a third axis, wherein the third axis passes through the RCM and is fixed relative to the base;
wherein the transmission mechanism comprises pulley assemblies arranged at the first joint and the second joint, and transmission assemblies connecting a dynamic source, the pulley assemblies, and the instrument holder, wherein each of the pulley assemblies comprises:
a flanged shaft, comprising a shaft and a flange fixed to each other, wherein the flange is connected to a respective one of the transmission assemblies, the shaft at the first joint is connected to the first link and is rotationally adjustable relative to the first link, and the shaft at the second joint is connected to the second link and is rotationally adjustable relative to the second link; and
a pulley, rotatably sleeved on the shaft and connected to a respective one of the transmission assemblies, wherein the pulley at the first joint is fixed to the second link, and the pulley at the second joint is fixed to the third link.

2. The robotic manipulator according to claim 1, wherein an end surface of the shaft is provided with a protrusion, wherein the first link and the second link each comprise a fastener and each define a connection groove corresponding to the protrusion, the protrusion extends into the connection groove, and the connection groove is larger in size than the protrusion, wherein the fastener is configured to fix and adjust a position of the protrusion in the connection groove.

3. The robotic manipulator according to claim 2, wherein the first link and the second link each further define an adjustment hole through which the fastener passes through, wherein the adjustment hole communicates with the connection groove, and the fastener extends into the connection groove through the adjustment hole to abut against the protrusion.

4. The robotic manipulator according to claim 3, wherein
the fastener comprises a first bolt, and an inner wall defining the adjustment hole is provided with internal threads matching external threads of the first bolt.

5. The robotic manipulator according to any one of claims 1 to 4, wherein the transmission assemblies comprise straps comprising a second strap and a third strap, wherein two ends of the second strap are respectively connected to the flange at the first joint and the pulley at the second joint, and two ends of the third strap are respectively connected to the flange at the second joint and the instrument holder.

6. The robotic manipulator according to claim 5, wherein the transmission mechanism further comprises a driving pulley coupled to the dynamic source and driven by the dynamic source to rotate, and the straps further comprise a first strap two ends of which are respectively connected to the driving pulley and the pulley at the first joint.

7. The robotic manipulator according to claim 6, wherein at least one of the first, second, and third straps has two discontinuous segments which are wound in opposite winding directions around the pulley or a driven wheel to drive the pulley or the driven wheel to rotate in two opposite directions, respectively.

8. The robotic manipulator according to claim 6 or 7, wherein at least one of the flange at the first joint, the flange at the second joint, and the driving pulley is provided with a tensioning structure configured to adjust tension of the straps.

9. The robotic manipulator according to claim 8, wherein the at least one tensioning structure comprises a chamber, a slot, and a bolt, wherein the chamber communicates outside through the slot, and a movable tensioning block is disposed in the chamber, wherein the bolt abuts against the tensioning block to restrict movement of the tensioning block; and
the strap has an end extending into the chamber through the slot and fixed to the tensioning block.

10. The robotic manipulator according to claim 9, wherein the bolt comprises a second bolt, wherein the flange has an inner wall defining a through hole communicating the chamber, the inner wall is provided with internal threads mating external threads of the second bolt, and the second bolt extends into the chamber through the through hole to abut against the tensioning block to adjust a position of the tensioning block.

11. The robotic manipulator according to claim 10, wherein the bolt comprises two second bolts, and the two second bolts respectively abut against two sides of the tensioning block to restrict movement of the tensioning block.

12. The robotic manipulator according to any one of claims 6 to 11, wherein the first strap, the second strap, and the third strap comprise steel belts.

13. A joint structure, comprising:
a flanged shaft, comprising a shaft and a flange fixed to each other;
a pulley, pivotally sleeved on the shaft;
a first pivot member, connected to the shaft, wherein the shaft is rotationally adjustable relative to the first pivot member; and
a second pivot member, fixed to the pulley, wherein the second pivot member is pivotable relative to the first pivot member,
wherein a flexible transmission assembly is provided inside the second pivot member, the flange is connectable to a rotatable member through the flexible transmission assembly, and the rotatable member rotates relative to the second pivot member with orientation adjustment of the shaft relative to the first pivot member.

14. The joint structure according to claim 13, wherein an end surface of the shaft is provided with a protrusion, wherein the first pivot member comprises a fastener and defines a connection groove corresponding to the protrusion, the protrusion extends into the connection groove, and the connection groove is larger in size than the protrusion, wherein the fastener is configured to fix and adjust a position of the protrusion in the connection groove.

15. The joint structure according to claim 14, wherein the first pivot member further defines an adjustment hole through which the fastener passes, wherein the adjustment hole communicates with the connection groove, and the fastener extends into the connection groove through the adjustment hole to abut against the protrusion.

16. The joint structure according to claim 15, wherein the fastener comprises a bolt with external threads, and an inner wall defining the adjustment hole is provided with internal threads matching the external threads of the bolt.

17. The joint structure according to any one of claims 13 to 16, wherein the pulley is provided with a tensioning structure comprising a chamber, a slot, and a bolt, wherein the chamber communicates outside through the slot, and a movable tensioning block is disposed in the chamber, wherein the bolt abuts against the tensioning block to restrict movement of the tensioning block, wherein the flexible transmission assembly extends into the chamber through the slot and is fixed to the tensioning block.

18. A surgical robot, comprising at least one robotic manipulator according to any one of claims 1 to 12, or comprising the joint structure according to any one of claims 13 to 17.
